# EUROPEAN PATENT SPECIFICATION

(11) **EP 0 970 244 B1**
(45) Date of publication and mention of the grant of the patent: **08.09.2004**
(21) Application number: 98908258.1
(22) Date of filing: 06.03.1998
(51) Int. Cl.: C12Q 1/68, B01L 3/00

(54) **A METHOD AND SYSTEM OF IDENTIFICATION OF A MEAT PRODUCT BY GENOTYPING**
VERFAHREN UND SYSTEM ZUR IDENTIFIZIERUNG EINES FLEISCHPRODUKTS MITTELS GENOTYPISIERUNG
PROCEDE ET SYSTEME D'IDENTIFICATION D'UN PRODUIT CARNE PAR ETABLISSEMENT DE GENOTYPE

(30) Priority: 07.03.1997 IE 970163
(43) Date of publication of application: 12.01.2000
(73) Proprietor: Parlanca Limited, Dublin 2 (IE)
(72) Inventor: MEGHEN, Ciaran Niall, Dalkey, County Dublin (IE); CUNNINGHAM, Edward Patrick, Dunboyne, County Meath (IE); BRADLEY, Daniel Gerard, Dublin 4 (IE); MacHUGH, David Evan, Dublin 18 (IE); LOFTUS, Ronan Thomas, Ballina, County Mayo (IE)
(74) Representative: Gordon, Naoise Padhraic Edward
(86) International application number: PCT/IE1998/000021
(87) International publication number: WO 1998/039475

(56) References cited:
- GEORGES M. ET AL.,: "DNA fingerprinting in domestic animals using four different minisatellite probes" CYTOGENT. CELL GENET., vol. 47, - 1988 pages 127-131, XP002073607
- LANNELUC I. ET AL.,: "Genetic analysis of fingerprints in Merinos d'Arles X Booroola Merino crossbred sheep" ANIMAL GENETICS, vol. 23, - 1992 pages 339-346, XP002073608
- HABERFELD A ET AL: "DNA FINGERPRINTS OF FARM ANIMALS GENERATED BY MICROSATELLITE AND MINISATELLITE DNA PROBES" ANIMAL GENETICS, vol. 22, no. 3, 1 January 1991, pages 299-305, XP000564626
- HILLEL J ET AL: "DNA FINGERPRINTS OF POULTRY" ANIMAL GENETICS, vol. 20, no. 2, 1 January 1989, pages 145-155, XP000565649
- ROSSEN L. ET AL.,: "Inhibition of PCR by components of food samples, microbial diagnostic assays and DNA-extraction solutions" INT. J. FOOD MICROBIOLOGY, vol. 17, - 1992 pages 37-45, XP002073609
- WEEDN V.W., The Department of Defense DNA Registry, ATLANTA MEDICINE, vol.67, pages 55-57, (1993)

## Description

The present invention relates to a method of identification and more particularly to a method of identifying or tracing meat products.

Health and safety considerations demand that the origins of food products should be transparent. In addition, consumers now demand and some countries now require that the origins of meat products should be traceable so that quality assurance audits and monitoring procedures can be effectively and reliably carried out.

Whilst being an important source of nutrition, meat and meat-based products can also be a dangerous source of infection and accordingly it is critical for economic reasons that consumer confidence in such products should be maintained.

At present various methods and combinations of methods are used in an attempt to ensure the identity and source of meat products. For example, the identity of beef, pigs and poultry on a batch or consignment basis is sometimes recorded using batch/consignment numbers applied to the batches/consignments from source, through the slaughter process to the consumer. The method is time consuming, cumbersome and requires considerable resource from farmers, processors and government or other agencies.

Administratively intensive methods exist to record the identity of beef products up to slaughter. For example, in many countries government agencies issue numeric or alpha-numeric codes to farmers who subsequently allocate such codes to calves bred by them. Generally, the allocated codes are inscribed on ear-tags applied to both ears of an animal shortly after birth. The code is then manually recorded on a card peculiar to the animal which can include a farmer's name and also the dam's code so that the origin of the calf can be traced. Generally, where artificial insemination is used, the identity of the sire will be unknown. The card can then be used as a passport-type document for that particular animal.

Various other data on an animal may also be recorded, e.g. the frequency of tuberculosis testing and other veterinary information. Such information can be forwarded to a government agency. However, generally, the identification and testing records are not or are not easily integrated.

Following rearing of an animal, e.g. two years for beef animals and six to eight years for a dairy animal, the animal is generally slaughtered at an approved slaughterhouse. The ear-tags are removed from the beast at slaughter and the code recorded. Similarly, the animal passport data may be recorded and logged. The appropriate government agency then notes that the beast corresponding to the code has been slaughtered.

More particularly, the carcass of a slaughtered animal is usually divided in two. Each side of the carcass may be provided with a tag by the slaughterhouse. However, in a slaughterhouse in which many animals are slaughtered sequentially, during further division or butchering of the two halves of an individual carcass, it becomes impracticable to trace the identity of each meat cut from that carcass to the original beast from which the meat has been derived.

In order to meet demands from retailers and consumers various methods have been proposed in order to monitor the identity of the carcass after slaughter. However, all such methods rely on the continued use of tags, labels, etc. Clearly, due to the potentially high number of meat cuts derivable from a single animal, such procedures are highly resource intensive, expensive and subject to high incidence of error.

Accordingly, a need exists for reliable and rapid methods and systems of identification for meat products in which the identity of the meat product is retrievable throughout the lifetime of the meat product.

M. Georges, A-S Leguarre, M. Castelli, R. Hanset & G. Vassart, Cytogenet Cell Genet, 1988, (47) (127 - 131) demonstrated the possibility of obtaining DNA fingerprints in a variety of species using an M13 - derived sequence, a polymer of Jeffreys' core sequence, the human and globin hypervariable region and a mouse probe related to the Drosophila Per gene.

An object of the invention is to provide a method and system of identification of meat products.

According to the invention there is provided a method of tracing the origin of a meat product according to claim 1.

Preferably, the animal is a domesticated farm animal. The known animal may be first genotyped shortly after dismemberment of the animal carcass. Alternatively, the animal is first genotyped between birth and slaughter of the animal.

The method of the invention is particularly suited to the identification of beef.

Preferably, the genotyping procedure comprises the steps of sampling the animal tissue, extracting genetic material from the animal tissue and carrying out a molecular genetic analysis on the extracted genetic material. Preferably, the genetic material comprises DNA. More preferably, the DNA is amplified following extraction. Most preferably, the amplification comprises a polymerase chain reaction.

Advantageously, the sample is selected from the group comprising hair roots, hide, buccal swabs, blood, muscle, bone and any internal organs.

Most preferably, the sample is selected from the group comprising swabs, hide, hair roots and muscle.

Advantageously, the sample comprises a unit volume or size. Suitably, sampling is carried out with a unit volume or size retrieving dedicated sampling device.

Advantageously, the DNA is extracted using alkali extraction. Suitably the alkali comprises NaOH and KOH. More preferably, the DNA is extracted by adding a unit volume of alkali to the sample, heating the sample to extract the DNA, and neutralising the alkali. Suitably, the alkali is neutralised with an acid. Advantageously the acid comprises HCl. Preferably, a dye is added to the sample to visualise the sample. More preferably, the dye comprises cresol red.

Suitably, the pH of the sample is at least pH 8.

Suitably, the sample tissue is placed in an identification cell. Preferably, the extraction is carried out in said identification cell. Preferably, the identification cell is part of a microtitre plate.

Advantageously, the identification cell is labelled with the identification of the animal or the meat following sampling. Alternatively, an identification cell microtitre plate reference number is recorded on a computer.

Alternatively, the label information is entered into a computer prior to extraction. Most preferably, a microtitre plate reference number is entered into a computer.

Advantageously, the information on the computer is searchable.

Preferably, the genotype of the genotyped meat is entered onto the computer. More preferably, the genotype is searchable. Most preferably, the genotype is encoded.

The invention also extends to a method for identifying the animal from which a meat product is derived comprising sampling the animal tissue, storing the sample, extracting genetic material from the sample as required, carrying out a molecular genetic analysis on the extracted genetic material and encoding the results of the molecular genetic analysis, entering the sample information and coded genetic analysis onto a computer database, the sample information and the encoded genetic analysis being searchable so that the molecular genetic analysis of a meat sample can be matched with the molecular genetic analysis of an animal.

The invention also relates to the use of molecular genetic analysis for identifying the animal from which a meat product is derived. Preferably, the molecular genetic analysis comprises a Polymerase Chain Reaction amplification.

Further, a microtitre plate holder comprising a microtitre plate holding means, identification codes for each well of the microtitre plate and controlling means for allowing selective access to the wells of the microtitre plate is provided.

Preferably, the controlling means comprises a covering means for selectively covering and revealing the wells of the microtitre plate.

Advantageously, the covering means comprises a door having at least one opening therein for selectively revealing or permitting access to a well, the door being slidable on the microtitre plate. Suitably, the door is slidably mounted in a frame mountable on the microtitre plate. Preferably, the opening comprises a circular opening to permit access to a single selected cell. More preferably, the at least one opening comprises a slot for permitting access to a row or column of cells.

Further a kit for identifying the animal from which a meat product is derived comprising reagents for extracting genetic material, a microtitre plate handling device and a means for recording the identity on the genetic material is disclosed. The kit optionally further comprises syringe means for transferring samples.

An advantage of the invention is that the identity of animal meat products can be retrieved and traced rapidly and at reasonable cost requiring minimal resource.

An application of genetic identification techniques, as a means of providing routine quality control in the beef industry is provided. The DNA based approach has several key advantages over any other system:
i) DNA identification represents a significant cost saving over any other approach.
ii) The DNA system does not require any significant alteration of existing production practices.
iii) Benefits of the system are available not only to large supermarket chains and other major buyers but are also accessible to smaller retailers and buyers.
iv) There are no negative food safety implications which contrasts sharply with any system that requires the physical labelling of meat in the production phase.
v) The system is subject to significantly less operator error and is tamper-proof.

The method of the invention requires that a sample is taken from every animal at a point in the production phase before the life-time identity of the animal has been lost. Depending on the particular practices this can be as the animals are first delivered to the meat plant or at the point where factory kill numbers are allocated to the carcasses. Sampling is technically straightforward and involves minimal interference with existing processing systems. The samples are submitted to the testing laboratory and prepared for analysis. This activity provides the framework against which meat traceability can be tested. Meat samples from any point after the carcass identity has been lost can be matched easily to the correct animal of origin through the method of the invention.

Samples need only be taken at a single point in the processing chain. This is typically at the point where the carcasses are batched for the retail client (storage cold room) - where a meat sample is taken from each carcass and labelled with the appropriate internal factory kill number or equivalent.

The method of the invention therefore provides for 100% meat-carcass traceability without the need for extensive DNA analysis. At any point into the future retail meat samples can be traced to the carcass of origin by virtue of the fact that meat samples have been taken from all source carcasses and kept in storage for the life of the meat product - typically 7 months; this includes the recommended maximum storage time for home-freezing of meat products.

In addition the invention provides that the retailer is able to independently verify the accuracy of label information at any point in the future. From a quality control perspective the retailer may wish to implement a routine of meat sampling in order to achieve levels of statistical confidence in the conformity of meat deliveries to a particular contract specification. The appropriate statistical model is the hypergeometric distribution but either the binomial or Poisson distribution give similar results.

Using the binomial distribution the following example indicates the level of retail sampling required in order to achieve various levels of confidence, where confidence is defined as the probability of detecting a non-traceable meat product.

| **To detect 1 out of specified carcass in** | **No. meat-carcass samples to test** **(p0.05)** | **No. meat-carcass samples to test** **(p0.01)** |
|---|---|---|
| 50 | 150 | 300 |
| 100 | 300 | 500 |
| 200 | 600 | 1000 |
| 300 | 900 | 1400 |
| 400 | 1200 | 1900 |

The method of the invention therefore adapts the genotype unique to every individual animal to a user friendly animal/meat tracing system. The genotype, in the form of DNA sequences, is replicated in every cell of an animal and is therefore to be found unchanged in every tissue of an animal carcass. A particular advantage of the exploitation of the DNA sequence is that the DNA sequence remains unchanged after death - and often even after cooking, curing or processing of meat products. Therefore, the integrity of the identification information of a beast is maintained from birth through to consumption.

In contradistinction, labelling and tagging methods cannot convey the identity of an animal through cooking, curing or processing procedures.

A further advantage of the invention is that the genotype of a beast is tamper proof - the genetic code cannot be modified throughout the lifetime of an animal or subsequently.

Accordingly, the present invention has adapted the in-built genetic identification code of every animal into a method for tracing the origins of an animal.

Considerable prejudice exists against the use of genetic information on a macro scale as compared with use of such methods on a micro scale as is employed in forensic science. More particularly, known DNA sampling, extraction and analysis procedures are regarded as highly resource and skill intensive operations.

However, the present invention overcomes such prejudice and provides a simple yet reliable method of sampling, extracting and analysing meat products on a macro scale that facilitates the application of molecular biology to meat tracing and identification.

The method of the invention may be made up of a number of procedural steps, namely, tissue sampling, extraction of genetic material from the sampled tissue, molecular genetic analysis of the genetic material, and where the tissue sample is taken from a meat product, comparison of the genotype with known animal genotypes stored on a database.

The invention also provides a sample handling system or regime that ensures continuity and integrity of each sample in the aforementioned method.

The invention will now be described having regard to the accompanying drawings and examples, in which:
Fig. 1 is a top plan view of a microtitre plate holder for use in the method and system of the invention;
Fig. 2 is an exploded view of the components of the microtitre plate holder of Fig. 1;
Fig. 3 is a perspective view from above of the microtitre plate holder of Figs. 1 and 2 with the top plate removed;
Fig. 4 is a side elevation of the microtitre plate holder with the top plate disposed over the microtitre plate and base plate, and
Fig. 5 is a side elevation of the microtitre plate holder with the top plate mounted on the microtitre plate and base plate.

Generally, suitable methods of the invention can comprise:

### Tissue Sampling

A tissue sample may be taken from an animal at any time in the lifetime of an animal but before the carcass identity is lost. Accordingly, the method and system of the invention can be used in substitution for current animal tag identification methods or in addition to such methods. For example, if it is believed that an animal tag identification method is satisfactory up to the point of slaughter, tissue sampling of an animal can be effected at a slaughterhouse prior to loss of carcass identity.

The tissue sample can comprise hair roots, hide, buccal swabs, blood, muscle or any internal organs. The tissue samples can also comprise bone although the aforementioned sample types are preferred.

The tissue sample is placed in an identification tube or cell which, where the method of the invention is used in combination with an animal tag identification system, is marked with the animal tag identification code.

The identity of the cell remains constant throughout the method of the invention thereby guaranteeing the integrity and continuity of the tissue sample during extraction and analysis. In contradistinction, forensic analyses carried out on a micro scale demand frequent changes in sample receptacles which can give rise to unreliability and experimental error.

The amount/size of sample required is determined by the subsequent steps used in the method of the invention and the specific methods of analysis used. Ideally, the size/volume of the tissue sample retrieved should be as consistent as possible from animal to animal.

Non-limiting examples of suitable sample sizes/methods include:

| | |
|---|---|
| Non-fatty meat | 0.0002g - 0.0010g |
| Hide | 0.0004g - 0.0010g |
| Hair roots | >5, <20 |
| Buccal swabs | 15 to 20 seconds of rubbing with modest pressure in the area between outer lip and gum using one Cytosoft (Trade Mark) cytology brush. |
| Bone | 0.0020g - 0.0040g |
| Blood | 30 to 70µl |

Typically, the identification cell is labelled using a numbering system bearing a code corresponding to the code on the identification tag of the animal.

The identification cell is then placed in a microtitre plate having a multiplicity of wells, e.g. 96 wells or multiples thereof. Each well is provided with a code.

The numbering system used in the method and system of the invention utilises the animal identification number as originally indicated on the animal ear-tag. A derivative of the animal identification number or a completely new number can also be used. Accordingly, the genotype of a particular animal is easily traceable at all times.

The microtitre plate, carrying the identification cells, is then used in a laboratory for extraction of the genetic material from the samples and analysis. Alternatively, the samples can be frozen for preservation and archived, for example, in the factory/slaughterhouse or a central storage location for future extraction/analysis as required.

A sampling device may be supplied to the farmer, a slaughterhouse or retailer. The sampling device preferably takes a consistent and reproducible sample from individual animals whilst simultaneously avoiding any cross-contamination of tissue. Accordingly, the size and volume of sample tissues derived from individual animals would be consistent.

An advantage of uniform sample volumes/sizes is that reagents used in the subsequent extraction and analysis processes (see below) could in turn be uniform and would not be subject to variation due to sample size/volume.

### Extraction of DNA

Extraction of DNA from tissue samples using standard techniques generally requires up to two days using known organic extraction methods. The forensic or micro scale organic extraction procedures also require use of hazardous chemicals and expensive reagents such as Proteinase K or phenol. In addition, known DNA extraction and transfer often requires use of multiple centrifugation steps.

A strong prejudice exists against the use of genetic analysis on a macro scale due to the labour intensive and time consuming extraction methods required to extract high purity DNA from tissue samples. For example, Proteinase K is employed to digest proteins present in the sample which could inhibit the amplification and analysis of the extracted DNA. Surprisingly, it has been found that DNA of sufficiently high quality for amplification and subsequent analysis can be rapidly extracted from animal tissue on a macro scale without requiring prolonged and repeated transfer and centrifugation steps and the use of a proteinases such as Proteinase K. The extracted DNA material can then be amplified using the polymerase chain reaction discussed further below.

Accordingly, heretofore a strong prejudice has existed against use of molecular genetic analysis on a routine macro scale due to the high quality of DNA required for amplification and subsequent analysis.

Many extraction methods known in the art can be used to extract genetic material from tissue samples in the present invention.

However, a preferred method of extraction in which complex protein digestion, transfer and centrifugation steps are not required for use in the method and system of the present invention which facilitates rapid extraction of DNA from tissue samples is outlined below. The preferred method comprises the use of an alkali extraction method employing NaOH. However as will be appreciated by those skilled in the art KOH could also be used. The alkali typically has a concentration from approximately 150mM to approximately 200mM. A preferred reagent is:

| | | |
|---|---|---|
| Solution A | 200mM | NaOH |
| | ^{~}0.5mM | Cresol Red |

No manipulation of the tissue sample prior to extraction is required. An operative in a laboratory enters the microtitre plate number/alpha-numeric code into a computer database and prior to handling each individual identification cell enters the alpha-numeric code on the identification cell label and the number of the corresponding microtitre plate well into the database. Thereafter, the principal field of reference on the database is the microtitre plate number, e.g. if an animal code is entered, the database directs an operative to a plate.

The volume of reagents used in the extraction is determined by the tissue type and the size/volume of the tissue sample. Typically, sample size/volume is determined on a unit volume basis with a typical unit volume being in the range of 25 microlitres to 100 microlitres.

A unit volume of 200mM NaOH (Solution A) is added to the tissue sample in the identification cell. The identification cell is then heated.

The temperature and period of heating should be sufficient to cause the double-stranded DNA material to revert to single strand form. Accordingly, temperatures in the range of from approximately 95°C to approximately 99°C are preferred and have been found to be particularly effective. The identification cell is typically heated in the aforementioned temperature range for a period of between approximately 15 minutes and approximately 25 minutes.

Heating at a temperature of 97°C for 20 minutes has been found to be particularly effective.

Solution A comprises a dye. The dye is selected to render the sample solution which is usually transparent coloured. The dye is selected so as not to react with the extracted DNA nor to interfere with the subsequent genetic analysis. A preferred dye is cresol red.

An advantage of cresol red is that it also acts as a pH indicator to show that the sample pH is in the correct range following mixture of Solution A with a second Solution (discussed further below) B.

An advantage of incorporation of cresol red into Solution A is that a colour change occurs upon combination of Solutions A and B, i.e. a purple to red colour change.

Sample pH is important as many molecular genetic analysis methods demand samples of genetic material having a specified pH. In the preferred analysis method outlined below a pH of approximately 8 to approximately 9 is preferred.

Cresol red has the following colour ranges:
pH 8-9: red
pH >9: purple
pH <8: yellow

Accordingly, cresol red is a highly suitable dye which also functions as a pH indicator.

The use of the dye in the identification cell facilitates rapid, easy and highly visible handling of samples on a macro scale.

Solution B comprises an acid to reduce the pH of the identification cell solution. Solution B typically comprises an acid such as TRIS HCl containing HCl at a concentration from about 150mm to about 250mm HCl. A preferred Solution B comprises:

| | |
|---|---|
| Solution B | 100mM TRIS HCl pH 8.5 |
| | 200mM HCl |

For analysis purposes, it is desirable that equal proportions of the TRIS HCl solution as compared with the NaOH first solution should be utilised.

A unit volume of Solution B is then added to the identification cell. The final solution therefore has a pH of approximately 8.5 based on TRIS HCl.

The sample handling techniques of the invention ensure that transfer of the sample between tubes is minimised. More particularly, the identity of the cell remains consistent throughout the entire method and system of the invention to guarantee continuity of samples.

As indicated previously, all transfers, conveying and analysis operations are carried out on a microtitre plate basis and not on a well or identification cell basis. Accordingly, in contradistinction with the forensic methods of the prior art the incidents of error are greatly reduced and loss of individual cells is practically eliminated.

Microtitre plates can be easily stored due to their comparatively small size and accordingly extracted genetic material can be stored in a freezer for prolonged periods of time.

The sample contained within each identification cell is overlaid with oil in order to preserve the sample. The samples can be frozen or analysed immediately as outlined below.

### DNA Amplification

The extracted DNA is amplified prior to DNA analysis.

A particularly preferred method of amplification is Polymerase Chain Reaction (PCR) analysis. As will be appreciated by those skilled in the art, PCR is an in-vitro method for enzymatically synthesising defined sequences of DNA. The reaction uses two oligonucleotide primers that hybridise to opposite strands and flank the target DNA sequence that is to be amplified.

A general guide to PCR analysis protocols is to be found in "PCR Protocols: A guide to methods and applications. Eds: M.A. Innis, D.H. Gelfand, J.J. Sninsky, J.J. White. San Diego, CA: Academic Press, 1990 which is incorporated herein by reference.

Generally in PCR amplification a repetitive series of thermal cycles involving template denaturation, primer annealing and extension of the annealed primers by Tag DNA polymerase results in exponential accumulation of specific short DNA sequences (see below).

The PCR process can therefore be used to detect length variation at DNA sequence repeats or microsatellites. PCR therefore can identify evenly distributed and polymorphic microsatellite markers in DNA samples. In the present invention, microsatellite loci peculiar to the species of animal being tested can be amplified and analysed.

The number of microsatellite loci required in the method and system of the invention is determined by the power of resolution required of the test method. For example, the number of microsatellite loci could be comparatively low where the method and system of the invention is employed on a batch basis while the number would be increased where the system and method are to be used for example on a national herd.

The International Society of Animal Genetics (ISAG) has approved nine microsatellite loci for use in bovine parentage verification and a further four markers have been selected for further study. The ISAG markers are suitable for use in the method and system of the present invention. The markers now form the basis of an internationally standardised marker set. These markers have been optimised as kits for use with an Perkin Elmer AB1377 Automated Sequencer (Trade Mark) and the Li-Cor 4200 Dual laser Sequencers (Trade Mark). These markers and subsets of these markers have been identified as being most suitable for use in beef traceability. Details of the markers are as follows:

### ISAG Markers Recommended for Use in Bovine Parentage Verification.

Marker Name (size range: base pairs)
Chromosome Primer Sequences (5'-3')

BM 1824 (178-190bp)
PRIMER 1: GAG CAA GGT GTT TTT CCA ATC
PRIMER 2: CAT TCT CCA ACT GCT TCC TTG

BM 2113 (125-143bp)
PRIMER 1: GCT GCC TTC TAC CAA ATA CCC
PRIMER 2: CTT CCT GAG AGA AGC AAC ACC

SPS 115 (240-262bp)
PRIMER 1: AAA GTG ACA CAA CAG CTT CTC CAG
PRIMER 2: AAC GAG TGT CCT AGT TTG GCT GTG

ETH 3 (117-129bp)
PRIMER 1: GAA CCT GCC TCT CCT GCA TTG G
PRIMER 2: ACT CTG CCT GTG GCC AAG TAG G

ETH 10 (210-226bp)
PRIMER 1: GTT CAG GAC TGG CCC TGC TAA CA
PRIMER 2: CCT CCA GCC CAC TTT CTC TTC TC

ETH 225 (140-156bp)
PRIMER 1: GAT CAC CTT GCC ACT ATT TCC T
PRIMER 2: ACA TGA CAG CCA GCT GCT ACT

TGLA 122 (130-164bp)
PRIMER 1: CCC TCC TCC AGG TAA ATC AGC
PRIMER 2: AAT CAC ATG GCA AAT AAG TAC ATA C

TGLA 126 (109-127bp)
PRIMER 1: CTA ATT TAG AAT GAG AGA GGC TTC T
PRIMER 2: TTG GTC TCT ATT CTC TGA ATA TTC C

TGLA 227 (78-104bp)
PRIMER 1:    CGA    ATT    CCA    AAT    CTG    TTA    ATT    TGC    T
PRIMER 2:    ACA    GAC    AGA    AAC    TCA    ATG    AAA    GCA

Markers Recommended for Further Study

INRA 23 (197-223bp)
PRIMER 1:    GAG    TAG    AGC    TAC    AAG    ATA    AAC    TTC
PRIMER 2:    TAA    CTA    CAG    GGT    GTT    AGA    TGA    ACT    C

SPS 113 (156-133bp)
PRIMER 1:    CCT    CCA    CAC    AGG    CTT    CTC    TGA    CTT
PRIMER 2:    CCT    AAC    TTG    CTT    GAG    TTA    TTG    CCC

MGTG 4B (134-164bp)
PRIMER 1:    GAG    CAG    CTT    CTT    TCT    TTC    TCA    TCT    T
PRIMER 2:    GCT    CTT    GGA    AGC    TTA    TTG    TAT    AAA    G

TGLA 53 (144-190bp)
PRIMER 1:    GCT    TTC    AGA    AAT    AGT    TTG    CAT    TCA
PRIMER 2:    ATC    TTC    ACA    TGA    TAT    TAC    AGC    AGA

As indicated above suitable microsatellite loci and PCR methods for use in amplifying and subsequently analysing same are described in relation to the ABI PRISM (Trade Mark) Automated Genotyping System available from Perkin Elmer (Trade Mark). In this system, 11 microsatellite loci are amplified and analysed using the Perkin Elmer Stockmarks (Trade Mark) for Cattle Paternity Bovine PCR Typing Kit. A detailed description of the kit is to be found in "Stockmarks (Trade Mark) for Cattle Paternity Bovine PCR Typing kit Protocol", Part No. 401917, Rev. A which is incorporated herein by reference. The combined accuracy of the microsatellite markers provides an average power of exclusion (EPR) of greater than 99.999%

In the method of the invention one or more duplicate microtitre plates is prepared. The original microtitre plate is archived. The duplicate plates are prepared such that each identification cell corresponds with the identification cell of the primary identification microtitre plate.

As will be appreciated by the person skilled in the art, the number of duplicate microtitre plates is determined by the number and combination of loci.

The presence of the cresol red dye within the sample is an indicator as to those cells which have been duplicated and those identification cells which have not.

Briefly, using reagents in the typing kit, DNA amplification is carried out on the DNA sample previously prepared using fluorescent tagged primers specific to the 11 microsatellite loci. The kit comprises all labelled and unlabelled primers, polymerase, reference bovine DNA, dNTPs, and buffers to test the animals at the 11 loci. The markers are two base pair repeat microsatellite loci. The primer pairs are premixed and the PCR conditions optimised so that the loci can be amplified in three tubes (two quadruplex reactions and one triplex reaction), and then electrophoretically analysed in a single lane. The protocol lists all the necessary materials and describes the procedures for conducting the analysis.

As indicated previously, analysis of the samples is closely coordinated with data entry. For example, a file is opened on a computer for each PCR analysis. Each analysis is allocated a name or number. Each well sample is coordinated with the data on the computer so that the integrity of each well of the microtitre plate is clearly maintained throughout the analysis.

Other microsatellite loci which are suitable for use in the method and system of the invention are the URB markers as developed by H. Lewin at the Bovine Blood Typing Lab, Saskatchewan Research Council, Saskatoon, Saskatchewan, Canada. The number of loci required is determined by the power required of the test.

### Molecular Genetic Analysis

Following PCR amplification of the samples, DNA fragment analysis is then carried out on the amplified genetic materials. An example of a suitable DNA analysis system is the ABI Prism 377 (Trade Mark) DNA Sequencer referred to above or alternatively the Li-Cor 4200S (Trade Mark).

A detailed procedure of a suitable analysis method is to be found in th aforementioned protocol.

Briefly, in present invention the above mentioned protocol is employed and typically, three reaction solutions resulting from PCR amplificatio are mixed and loaded onto an electrophoretic gel. A sharp tooth comb is used for loading. Preferably, a multi-channel Hamilton syringe is used to load the gel.

The results of the analysis are presented in sequence gel format or colour coded electropherograms which are unique to individual animals. The data is then transformed into coded genotypes unique to an animal.

Accordingly, the genetic profile of a tissue sample can be searched on the database to locate a match relating to the original animal from which the tissue derived thereby facilitating rapid identification of the origins of the meat product.

Clearly, the system and method of the invention is adapted for use with any of other known molecular genetic analysis methods e.g. matrix assisted laser desorption ionisation combined with time of flight analysis of ejected ions (MALDI-TOF) techniques. In addition, molecular genetic analysis methods under development could also be substituted for the PCR analysis referred to above. An example of such a method is the Affymetrix GeneChip (Trade Mark) array. An advantage of the microchip method is that electrophoresis is not required. Alternatively single nucleotide polymorphisms (SNP's) could be used as they become available.

### Sample Handling Method and Device

In order to enhance the use of genotyping techniques for identifying the animal from which a meat product is derived, a unique sample handling method and device can be employed.

The use of an identification cell as previously described helps to ensure continuity in the identification of the sample using the method and system of the invention. In addition, the use of a dye in order to aid visualisation of the sample in the method and system of the invention further minimises experimental error and facilitates a rapid throughput.

In addition, the use of microtitre plates, as previously described, ensures rapid location, throughput and storage of samples.

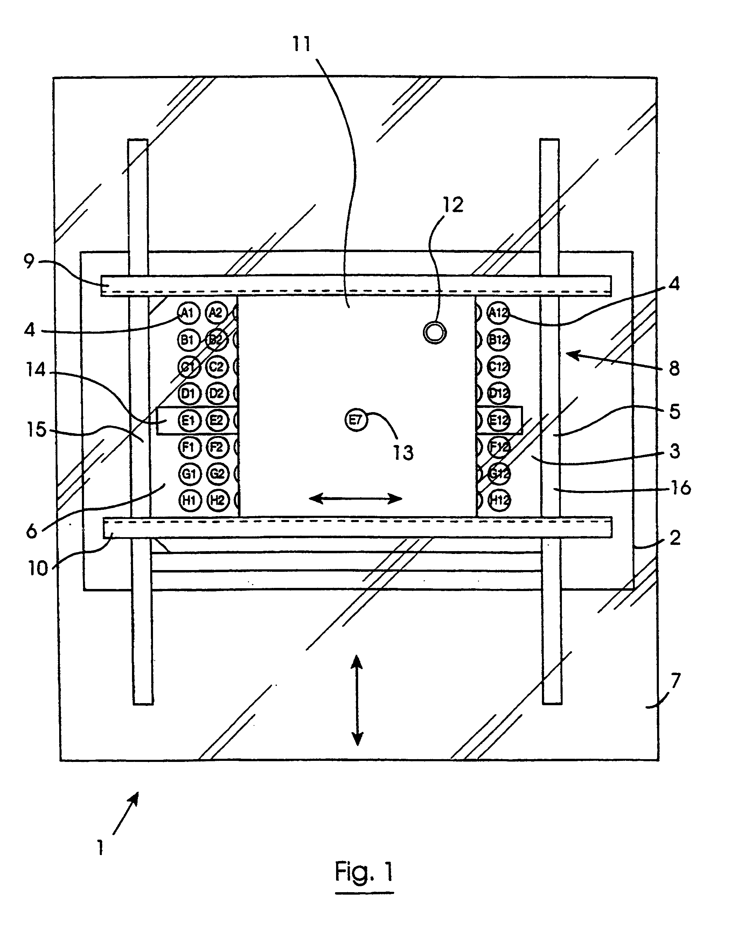

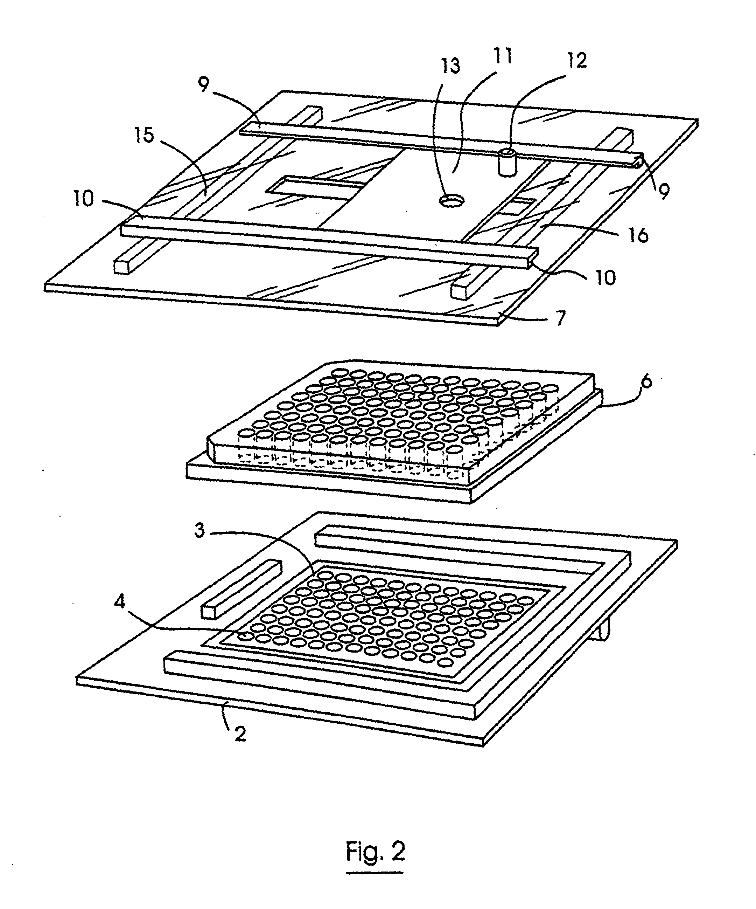

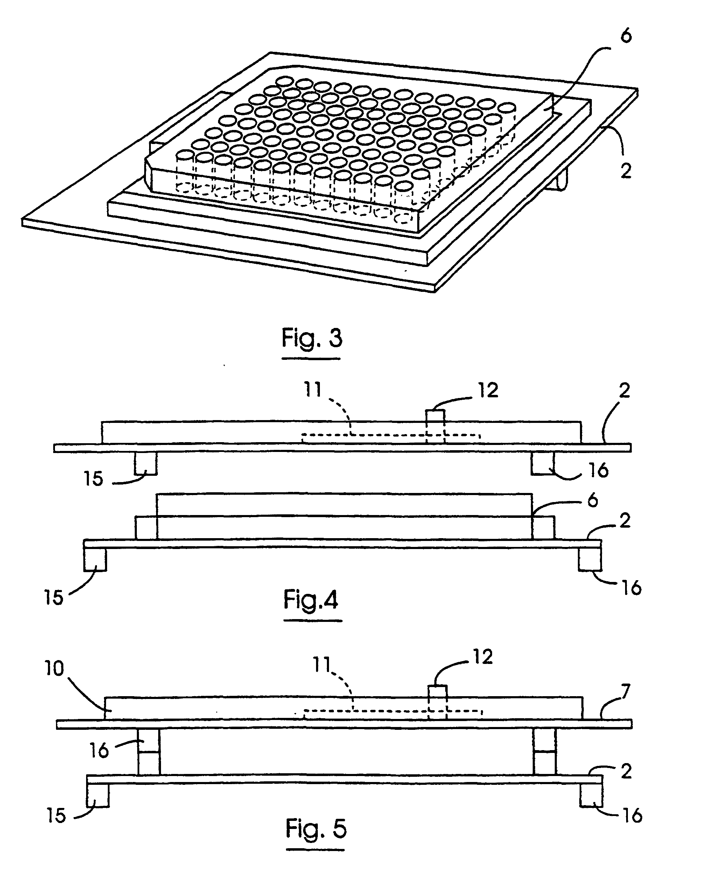

Figs. 1 to 5 show a microtitre plate holder 1 which can be used to hold a microtitre plate 6 during the process in accordance with the invention.

As shown in the drawings, the microtitre plate holder 1 is made up of a transparent plastics base plate 2 having a template 3 printed or mounted thereon. The template 3 is made up of a series of cell numbers 4 corresponding to the individual cells of a microtitre plate 6. As indicated previously, the microtitre plate 6 can have 96 wells therein or multiples thereof. A microtitre plate holding frame 5 is formed on the upper surface of the base plate 2 to receive the microtitre plate 6 therein. As shown in Fig. 1, the microtitre plate 6 is disposed over the template 3 in the holding frame 5 so that the individual wells of the microtitre plate 6 correspond to the cell numbers 4 printed on the template 3.

A top plate 7, also of transparent plastics material, is mounted over the microtitre plate 6. The top plate 7 has a mounting frame 8 on its underside to engage the microtitre plate 6 at its sides. The mounting frame 8 is made up of two side members 15,16 which are in sliding relationship with the sides of the microtitre plate 6 so that the top plate 7 is slidable over the microtitre plate 6 in a horizontal manner in the direction indicated by the arrows in Fig. 1.

The top plate 7 is further provided with an upper runner 9 and a lower runner 10 on its top surface. The upper and lower runners 9,10 are parallel and are adapted to receive a door 11 between the upper and lower runners 9,10. The door 11 is slidable from left to right between the upper and lower runners 9,10.

The door 11 is provided with a handle 12 and a well hole 13.

The well hole 13 is positioned over a horizontal elongated slot 14 defined in the top plate 7. Accordingly, movement of the door 11 between the upper and lower runners 9,10 displaces the well hole 13 along the length of the slot 14.

The top plate 7 and the door 11 are formed from tinted or blackened plastics so that only the selected well of a microtitre plate 6 disposed beneath the well hole 13 will be immediately visible to the eye.

The microtitre plate holder 1 can be used in the method and system of the invention in a number of ways.

For example, during transfer of samples from sample tubes to microtitre plates, a microtitre plate 6 is mounted in a holder 1 as previously described. The top plate 7 is located over the microtitre plate 6 so that the slot 14 is disposed over the first row of wells of the microtitre plate 6. The door 11 is then positioned using the handle 12 by an operative so that the well hole 13 is disposed over the first well of the first row. A first sample is then placed in the first well through the well hole 13 and the door slid between the upper and lower runners 9,10 to move the well hole 13 to the subsequent well hole. Accordingly, an operative is reminded at each stage during the transfer as to the correct well hole into which a sample is to be placed thereby eliminating experimental error.

The top plate 7 and the door 11 are adjusted as required in order to effect a transfer to the remaining wells of the microtitre plate 6. Due to the blackened or tinted nature of the door and the top plate 7 only the well beneath the well hole 13 is immediately apparent to an operative in use. The holder 1 can also be adapted for use during the preparation of duplicates as previously described in relation to molecular genetic analysis. For example, during the preparation of duplicates from a microtitre plate 6 having twelve columns and eight rows, the door 11 can be removed from the top plate 7 and the top plate 7 disposed on the microtitre plate 6 as previously described. However, in the present case, a multi-channel pipette e.g. made up of twelve channels can be disposed over the slot 11 so that duplicates can be prepared on a row by row basis by moving the top plate 7 along the columns and rows in the direction indicated by the arrow in Fig. 1. Accordingly, errors are further eliminated during the duplication step as it is ensured that the operative removes samples from the correct row in the correct sequence.

Finally, during loading of an electrophoretic gel during molecular genetic sequence analysis, the holder 1 of the invention can be further adapted in order to provide rapid gel loading. More particularly, the holder 1 in combination with a multi-channel Hamilton syringe and the code typically used in electrophoretic analysis facilitates rapid and accurate application of sample from the microtitre plate 6 to the electrophoretic gel.

More particularly, the holder 1 is adapted by removing the door 11 from the top plate 7. The top plate 7 is then removed from the microtitre plate 6 and disposed such that the slot 14 is oriented in a vertical disposition, i.e. along the columns of the microtitre plate 6.

The multi-channel Hamilton syringe is provided with eight channels or syringes spaced apart a distance corresponding to every fourth comb space. Accordingly, in use, an operative orients the top plate with the slot disposed in a vertical disposition so that samples from well numbers A1, B1, C1, E1, F1, G1 and H1 are loaded in every fourth comb space. The top plate 7 is then displaced to the right so that well numbers A2, B2, C2, etc. appear in the slot. Accordingly, an operative removes samples from well numbers A2 to H2 with the multi-channel Hamilton syringe and loads the sample into the next comb space adjacent the preceding comb space. The process is repeated until all samples have been taken and the comb loaded.

Accordingly, this sample handling technique ensures sample identity continuity whilst also allowing rapid loading of electrophoretic gels using the device, Hamilton syringe and comb in combination.

The method and system of the invention will now be described having regard to the following examples:

### Example 1

Generalised Example Procedure
1. Animals for which meat traceability is required are sampled. A biological tissue sample (e.g. hair, blood, hide, etc.) is taken from each individual animal and linked to the animals tag number.
2. Samples are dispatched to a laboratory.
3. At the laboratory the animal ear-tag numbers are recorded in a computer database and samples are exposed to a DNA extraction procedure (e.g. alkali denaturation-neutralisation).
4. DNA samples are exposed to a polymerase chain reaction (PCR) developed specifically for individual identification.
5. The PCR products are assayed using an automated DNA sequencer.
6. DNA profiles, linked to the tag number, are recorded automatically in an "ANIMAL" computer data file.
7. DNA profiles are sorted into appropriate categories (i.e. by date, by abattoir, by contract, etc.) and are search ready.
8. Meat for which traceability is required is sampled.
9. Samples are dispatched to the laboratory and are checked into a "MEAT" data file.
10. At the laboratory samples are exposed to a DNA extraction procedure (e.g. alkali denaturation-neutralisation).
11. DNA samples are exposed to a polymerase chain reaction developed specifically for individual identification.
12. PCR products are assayed using an automated DNA sequencer.
13. DNA profiles are recorded automatically in the computer data file.
14. DNA profiles are compared against "ANIMAL" profiles and matches recorded. The individual from which the meat was derived is identified.
15. Results of the match search are reported.

### Example 2

Specific Example Procedure
1. All animals slaughtered in a specific meat plant are sampled. A biological tissue sample (e.g. hair, blood, hide, etc.) is taken from each individual animal and linked to the animals tag number.
2. Samples are dispatched to a laboratory.
3. At the laboratory the animal ear-tag numbers are recorded in a computer database and the samples are exposed to a DNA extraction procedure (e.g. alkali denaturation-neutralisation).
4. DNA samples are exposed to a polymerase chain reaction developed specifically for individual identification.
5. PCR product are assayed using an automated DNA sequencer.
6. DNA profiles, linked to the tag number, are recorded automatically in a "MEAT PLANT" computer data file.
7. DNA profiles are sorted into appropriate categories (i.e. by date, by consignment, etc.) and are search ready.
8. Direct or indirect customers of the specific meat plant sample meat product.
9. Samples are dispatched to the laboratory and are checked into a "MEAT PLANT CUSTOMER" data file.
10. At the laboratory samples are exposed to a DNA extraction procedure (e.g. alkali denaturation-neutralisation).
11. DNA samples are exposed to a polymerase chain reaction developed specifically for individual identification.
12. PCR products are assayed using an automated DNA sequencer.
13. DNA profiles are recorded automatically in the computer data file.
14. DNA profiles are compared against "MEAT PLANT" profiles and matches recorded. The individual from which the meat was derived is identified.
15. Results of the match search are reported.

### Example 3

### Comparative Data

The method and system of identification of the invention was compared with a known method of meat identification.

The known method is based on maintaining documented records of the origins of meat by the retailer. The paper tracing of the meat attempts to facilitate identification of individual meat cuts from a retail counter to the farm of origin. Generally, using the known method, meat tracing was carried out on meat products handled by the retailer and the meat supplier to the retailer. In order to facilitate the traceability, dedicated production procedures and technique were required. More particularly, at a meat plant, upon delivery of animals, appropriate recorded carcasses are selected for the retailer, boned and placed on a dedicated production line - the carcasses being grouped according to farm of origin and typically boned two carcasses at a time. Finished primal cuts of meat are then labelled according to the farm of origin and packaged.

The method of tracing of the invention was compared with the paper intensive method requiring a dedicated production of the prior art.

### Materials and Methods

### Sample Collection

Hair samples were collected from each of 326 cattle in the lairage of a meat supplier. Each sample was introduced into an envelope and labelled with the relevant ear-tag number for each animal.
The animals were then slaughtered. Subsequently, meat samples were collected from each of 28 carcasses destined for Retailer A. The meat samples were taken by cutting a small piece of trim from the neck region of the carcass. The samples were then labelled with a factory kill number. A factory kill sheet was employed to align the animal ear-tag number to the relevant kill number.

Following delivery of the butchered meat to Retailer A, meat samples were taken from the cold stores of the retailer. Samples were taken from each of the following meat cuts.

| | | |
|---|---|---|
| 8 | X | vacuum packed primal striploins |

Meat samples were taken from the cold stores at a second outlet of Retailer A. Samples were taken from each of:

| | | |
|---|---|---|
| 10 | X | vacuum packed primal briskets |
| 6 | X | vacuum packed primal rumps |

and were labelled with the Farm No. as indicated on the packaging. Quality control data was available to ensure that all meat sampled was from the initial kill referred to above.

Additional meat samples from the outlets of retailers unrelated to Retailer A and which did not operate the method of the prior art were also taken as follows:

| Unrelated Outlet #1 | | |
|---|---|---|
| 1 | X | Sirloin |
| 3 | X | Stewing Steak (taken from shop-front display) |

| Unrelated Outlet #2 | | |
|---|---|---|
| 1 | X | Aberdeen Angus (AA) Sirloin Steak |
| 2 | X | AA Mince (each sample taken from a single retail package) |
| 2 | X | Diced Beef (each sample taken from a single retail package) |
| 1 | X | T-bone Steak |
| 1 | X | Sirloin Steak |
| 1 | X | Rib Steak |
| 1 | X | Minute Steak |
| 2 | X | Low Grade Mince (each sample taken from a single retail package) |
| 2 | X | AA Stewing Steak (each sample taken from a single retail package) |
| 1 | X | Round Steak |

| Unrelated Outlet #3 | | |
|---|---|---|
| 1 | X | AA Sirloin |
| 1 | X | Round Steak |
| 1 | X | Minute Steak |
| 3 | X | Diced Steak (each sample taken from a single retail package) |
| 3 | X | Mince (each sample taken from a single retail package) |
| 1 | X | Sirloin |
| 1 | X | AA Striploin |
| 1 | X | Quick Fry Steak |

### DNA Extraction

For all of the meat samples the extraction protocol described above was used.

### DNA Amplification and Analysis

Each of the extracted meat samples, negative extraction, PCR and positive controls were amplified and analysed as previously

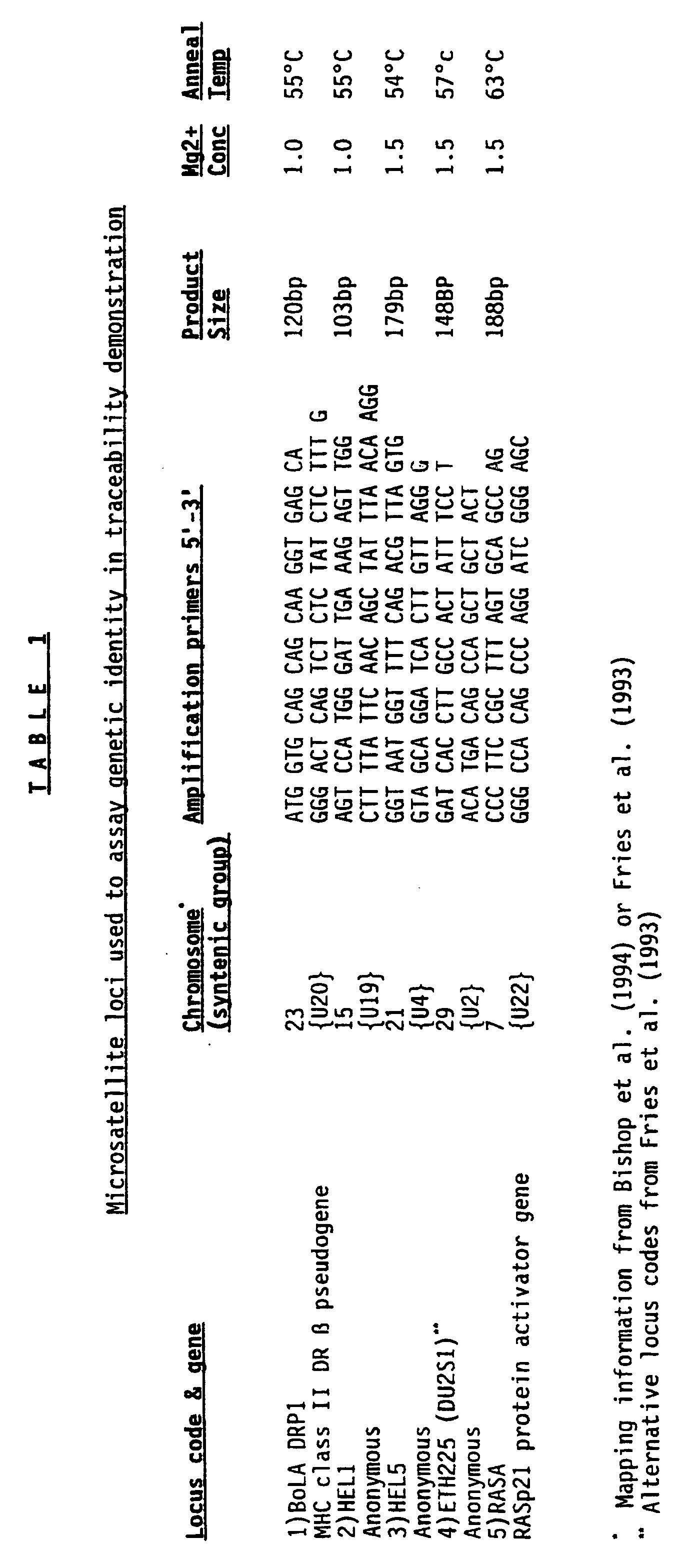

described. However, in the present example only 5 polymorphic microsatellites (Table 1) were required due to the comparatively small sample and the accordingly reduced power required. The amplication and analysis was carried under the following conditions:
PCR reactions were performed using 96-well microtitre plates with 5ng template DNA in 11µL reaction volumes using 0.5U of Taq polymerase with reaction buffer comprising 50mM KCl; 10mM Tris-HC] pH 9.0; 1.0-2.5mM MgCl₂ (see Table 1); 1% Triton X-100 (Trade Mark); 200µM dATP, dGTP, dTTP; 10µM dCTP. 0.3µM of each primer was added as was 0.5µCi (a-32P) dCTP. A 10µl mineral oil overlay was then added and amplifications were performed on a Hybaid Omnigene Thermal Cycler (Trade Mark) using a 4 min denaturation step at 94°C followed by 35 cycles of 30 sec at 94°C, 30 sec at 55-65°C (see Table 1), 30 sec at 72°C and a final extension step at 72°C for 4 min. Samples were then mixed with 10µL formamide loading solution. After heat treatment at 93°C for 4 min 1µL of the mixture was loaded on a 6% denaturing polyacrylamide gel. Following standard autoradiography genotypes were scored by reference to previously sized DNA standards and entered in the computer.

### Statistical Methodology

In order to determine the probability that two identical meat profiles may have occurred by chance (match probability), standard population genetic statistics were employed. Using a cattle DNA database of allele frequencies from previous studies it was possible to calculate match probabilities using the product rule. The product rule was considered appropriate given that each of the five loci used had previously been shown to be in Hardy-Weinberg equilibrium.

As the exact breed of each sample was unknown allele frequencies from a pooled British Isles (BI) population, a Simmental (SIM) population and a pooled European (EU) were used. It was considered reasonable that the true match probabilities for the experimental samples would fall within the range of probabilities represented by these three reference data-sets.

### Results

In total 82 samples were analysed for each of the five loci. Out of a possible 410 genotypes 93.4% or 383 were successfully scored. Only those samples in which all possible genotypes were scored, of which there were 67, were used for comparative analyses. 7 samples were scored for 4 loci, 4 samples for 3 loci and 4 samples for only two loci.

A pair-wise comparison of all fully scored samples revealed 100% consistency between the DNA results and the quality control data of the prior art for the samples. In every case the DNA results allowed individual meat samples to be traced back to the farm of origin. However the method and system of the present invention also allowed the exact animal of origin to be identified.

Accordingly, the method of the invention exploits the identity information encoded by DNA to facilitate complete traceability of meat products.

### The results are summarised as follows:

15 out of the 18 Retailer A meat samples, for which all five loci were scored, were traced to 11 individual animal profiles, all of which were within the pool of 28 carcasses sampled and all of which were from the correct farm of origin as provided for by the Retailer A paper system.

The three samples for which no matching carcasses were found were believed to be due to incomplete sampling of the carcasses.

12 Retailer A meat samples were found to have matching duplicates. In all cases duplicates were from the same type of cut and were from the correct farm of origin as provided for by the paper system of recordal of the prior art. This simply indicates that both rumps, briskets or striploins of the same animal were sampled and analysed. This is highly probable given that under the system of Retailer A the meat product from just two animals is boxed together.

None of the Retailer A meat samples or the carcass samples matched any of the non-Retailer A meat samples.

Between the non-Retailer A samples no matches were found. However within each of the individual retail sample sets some matching was evident. In unrelated outlet #2, the sirloin sample matched the rib steak sample and the two AA mince samples were found to be identical. In unrelated outlet #1 stewing steak II and III matched each other but did not match I. In unrelated outlet #3 all three mince samples were found to be identical.

All 28 carcass profiles were found to be unique to this study.

### Match Probabilities

Match probabilities were calculated for each of the 11 individual carcasses that matched Retailer A meat profiles (Table 2 and Table 3). Probability estimates were generated based on the allele frequencies for each of three populations i) BI - a combined population consisting of 33 Aberdeen Angus, 34 Hereford, 34 Jersey and 40 Kerry cattle ii) SIM - based on a population of 36 pure bred Simmental and iii) EU - a combined population of (i) and (ii) above plus 36 pure-bred Charolais and 40 pure-bred Friesian.

**TABLE 2**

| Match probabilities for each of the 11 carcasses | | | |
|---|---|---|---|
| **Animal No.** | **BI** | **SIM** | **EU** |
| A1 | 1.70E-06 | 5.87E-07 | 9.50E-07 |
| B1 | 4.71E-07 | 4.51E-09 | 4.00E-07 |
| C1 | 2.96E-06 | 3.77E-06 | 3.48E-06 |
| D1 | 1.07E-05 | 4.65E-07 | 9.15E-06 |
| E1 | 1.09E-10 | 5.91E-07 | 5.01E-08 |
| F1 | 9.79E-07 | 4.31E-10 | 5.44E-07 |
| G1 | 6.57E-06 | 4.41E-06 | 3.27E-06 |
| H1 | 1.99E-08 | 1.71E-07 | 2.51E-07 |
| I1 | 7.21E-07 | 3.84E-06 | 9.25E-07 |
| J1 | 1.49E-06 | 4.56E-07 | 3.06E-06 |
| K1 | 2.09E-07 | 7.26E-07 | 4.87E-07 |

**TABLE 3**

| Match probabilities sorted into lowest and highest | | | |
|---|---|---|---|
| **Animal No.** | **Low** | | **High** |
| A1 | 1.70E-06 | 9.50E-07 | 5.87E-07 |
| B1 | 4.71E-07 | 4.00E-07 | 4.51E-09 |
| C1 | 3.77E-06 | 3.48E-06 | 2.96E-06 |
| D1 | 1.07E-05 | 9.15E-06 | 4.65E-07 |
| E1 | 5.91E-07 | 5.01E-08 | 1.09E-10 |
| F1 | 9.79E-07 | 5.44E-07 | 4.31E-10 |
| G1 | 6.57E-06 | 4.41E-06 | 3.27E-06 |
| H1 | 2.51E-07 | 1.71E-07 | 1.99E-08 |
| I1 | 3.84E-06 | 9.25E-07 | 7.21E-07 |
| J1 | 3.06E-06 | 1.49E-06 | 4.56E-07 |
| K1 | 7.26E-07 | 4.87E-07 | 2.09E-07 |

From Table 3 it can be see that the highest probability was observed for carcass D1, where there was a one in 107,000 chance that the meat sample carrying the same profile may have been doing so by chance alone. The lowest probability was found to be for F1 where the likelihood that the Retailer A meat sample matched the carcass by chance was less than 1 in 40 billion.

Clearly, an extremely low possibility of accidental match probabilities exists with the method and system of the invention. Moreover, risks of an accidental match can be further reduced by increasing or varying the number of microsatellite loci.

### Discussion

The results demonstrate that the method and system of the invention is a highly effective quality indicator in meat processing and retailing.

The 5 locus marker set in this study was chosen on the basis that population data were available for comparative purposes. However, as indicated above, a number of other marker sets could be equally well suited to this particular application.

The method of the invention therefore facilitates the use of DNA as an internal product label in meat processing and retailing.

The method of the invention offers total traceability of meat products at reasonable cost. The genetic information is replicated in every cell of an animal and is therefore to be found unchanged in every fragment of a carcass. The genetic information is also tamper proof. Accordingly, the information cannot be modified at any stage to alter the identity of a meat product.
The procedure of the invention is simple to use and requires only small amounts of sample. The method and system of the invention also has applications in recording national herds, history of animals and movements of animals. The method of the invention negates the risk of tag switching or fraud and facilitates easy correction of lost tags or errors. Accordingly, the method of the invention can provide a consumer with the assurance that meat to be consumed has a transparent origin which can be easily derived and ascertained.

The method of the invention can also be used to identify meat on demand, e.g. in response to specific meat queries.

## Claims

1. A method of tracing the origin of a meat product comprising sampling animal tissue before the life-time identity of an animal is lost, storing the sample for the life-time of the meat product, genotyping the meat product and stored samples, and comparing the meat-product genotype with the stored-sample genotypes to identify the origin of the meat product.

2. A method as claimed in Claim 1 **characterised in that** the animal is a domesticated farm animal.

3. A method as claimed in Claim 1 or Claim 2 **characterised in that** the animal is sampled at a single point in the processing chain.

4. A method as claimed in Claim 3 **characterised in that** tissue sampling is effected at a slaughterhouse.

5. A method as claimed in Claim 3 or Claim 4 **characterised in that** tissue sampling is effected shortly after dismemberment of the animal carcass.

6. A method as claimed in any of Claims 3 to 5 **characterised in that** tissue sampling is effected at the point where carcasses are batched for a retail client.

7. A method as claimed in Claim 6 **characterised in that** a meat sample is taken from each carcass and labelled with an internal factory kill number or equivalent.

8. A method as claimed in claims 1 to 4 **characterised in that** the meat product is a beef product.

9. A method as claimed in any of claims 1 to 7 comprising sampling the animal tissue, extracting genetic material from the animal tissue and carrying out a molecular genetic analysis on the extracted genetic material.

10. A method as claimed in Claim 9 **characterised in that** the genetic material comprises DNA, the DNA is amplified following extraction and the amplification comprises a polymerase chain reaction.

11. A method as claimed in any of Claims 1 to 10 **characterised in that** the sample is selected from the group comprising hair roots, hide, buccal swabs, blood, muscle, bone and any internal organs and preferably the sample comprises a unit volume or size and sampling is carried out with a unit volume or size retrieving dedicated sampling device.

12. A method as claimed in Claim 10 or Claim 11 **characterised in that** the DNA is extracted using alkali extraction, the alkali comprises NaOH or KOH and the DNA is extracted by adding a unit volume of alkali to the sample, heating the sample to extract the DNA, and neutralising the alkali and the alkali is neutralised with an acid.

13. A method as claimed in Claim 12 **characterised in that** the acid comprises HC1.

14. A method as claimed in any of Claims 9 to 13 **characterised in that** a dye is added to the sample to visualise the sample and the dye comprises cresol red.

15. A method as claimed in any of Claims 12 to 14 **characterised in that** the pH of the sample is at least pH 8.

16. A method as claimed in any of Claims 9 to 15 **characterised in that** the sample tissue is placed in an identification cell and the extraction is carried out in said identification cell.

17. A method as claimed in Claim 16 **characterised in that** the identification cell is labelled with the identification of the animal or the meat following sampling and an identification cell microtitre plate reference number is recorded on a computer and the reference number on the computer is searchable.

18. A method as claimed in any of Claims 1 to 17 **characterised in that** the genotype of the genotyped meat is entered onto a computer and the genotype is searchable.

19. A method as claimed in Claim 18 **characterised in that** the genotype is encoded.

## Patentansprüche

1. Ein Verfahren zur Rückverfolgung des Ursprungs eines Fleischproduktes, das die Probenahme von Tiergewebe vor dem Verlust der Lebensdauer-Identität eines Tieres, das Aufbewahren der Probe für die Lebensdauer des Fleischproduktes, das Bestimmen des Genotyps des Fleischproduktes und der aufbewahrten Proben und das Vergleichen des Genotyps des Fleischproduktes mit den Genotypen der aufbewahrten Proben zur Identifizierung des Ursprungs des Fleischproduktes beinhaltet.

2. Verfahren gemäß Anspruch 1, **dadurch gekennzeichnet, dass** das Tier ein domestiziertes Nutztier ist.

3. Verfahren gemäß Anspruch 1 oder Anspruch 2, **dadurch gekennzeichnet, dass** eine Probe des Tieres an einem einzigen Punkt in der Verarbeitungskette genommen wird.

4. Verfahren gemäß Anspruch 3, **dadurch gekennzeichnet, dass** die Gewebeprobenahme in einem Schlachthof vollzogen wird.

5. Verfahren gemäß Anspruch 3 oder Anspruch 4, **dadurch gekennzeichnet, dass** die Gewebeprobenahme kurz nach der Zerstückelung des Tierschlachtkörpers vollzogen wird.

6. Verfahren gemäß einem der Ansprüche 3 bis 5, **dadurch gekennzeichnet, dass** die Gewebeprobenahme an dem Punkt vollzogen wird, an dem die Schlachtkörper für einen Handelskunden gestapelt werden.

7. Verfahren gemäß Anspruch 6, **dadurch gekennzeichnet, dass** von jedem Schlachtkörper eine Fleischprobe genommen und mit einer betriebsinternen Tötungsnummer oder einem Äquivalent gekennzeichnet wird.

8. Verfahren gemäß Ansprüchen 1 bis 4, **dadurch gekennzeichnet, dass** das Fleischprodukt ein Rindfleischprodukt ist.

9. Verfahren gemäß einem der Ansprüche 1 bis 7, das die Probenahme von Tiergewebe, das Extrahieren von genetischem Material aus dem Tiergewebe und das Durchführen einer molekular-genetischen Analyse des extrahierten genetischen Materials beinhaltet.

10. Verfahren gemäß Anspruch 9, **dadurch gekennzeichnet, dass** das genetische Material DNA beinhaltet, die DNA nach der Extraktion vervielfacht wird und die Vervielfachung eine Polymerase-Kettenreaktion beinhaltet.

11. Verfahren gemäß einem der Ansprüche 1 bis 10, **dadurch gekennzeichnet, dass** die Probe aus der Gruppe, die Haarwurzeln, Tierhaut, Wangenabstriche, Blut, Muskel, Knochen und beliebige innere Organe beinhaltet, ausgewählt ist und die Probe vorzugsweise eine Einheit Volumen oder Größe beinhaltet, und die Probenahme mit einer Probenahme-Vorrichtung, die zum Einholen einer Einheit Volumen oder Größe speziell entworfen ist, durchgeführt wird.

12. Verfahren gemäß Anspruch 10 oder Anspruch 11, **dadurch gekennzeichnet, dass** die DNA unter Verwendung von Alkaliextraktion extrahiert wird, das Alkali NaOH oder KOH beinhaltet und die DNA extrahiert wird, indem eine Einheit Volumen von Alkali zu der Probe hinzugegeben wird, die Probe erhitzt wird, um die DNA zu extrahieren, und das Alkali neutralisiert wird, und das Alkali mit einer Säure neutralisiert wird.

13. Verfahren gemäß Anspruch 12, **dadurch gekennzeichnet, dass** die Säure HCl beinhaltet.

14. Verfahren gemäß einem der Ansprüche 9 bis 13, **dadurch gekennzeichnet, dass** ein Farbstoff zu der Probe hinzugegeben wird, um die Probe zu veranschaulichen, und der Farbstoff Cresolrot beinhaltet.

15. Verfahren gemäß einem der Ansprüche 12 bis 14, **dadurch gekennzeichnet, dass** der pH der Probe mindestens pH 8 ist.

16. Verfahren gemäß einem der Ansprüche 9 bis 15, **dadurch gekennzeichnet, dass** das Probengewebe in eine Identifizierungszelle platziert wird und die Extraktion in der Identifizierungszelle durchgeführt wird.

17. Verfahren gemäß Anspruch 16, **dadurch gekennzeichnet, dass** die Identifizierungszelle mit der Identifizierung des Tieres oder des Fleisches nach der Probenahme gekennzeichnet wird und eine Referenznummer für die Mikrotiterplatte der Identifizierungszelle in einem Computer aufgezeichnet wird und die Referenznummer im Computer absuchbar ist.

18. Verfahren gemäß einem der Ansprüche 1 bis 17, **dadurch gekennzeichnet, dass** der Genotyp des genotypisch bestimmten Fleisches in einen Computer eingegeben wird und der Genotyp absuchbar ist.

19. Verfahren gemäß Anspruch 18, **dadurch gekennzeichnet, dass** der Genotyp codiert wird.

## Revendications

1. Un procédé de traçage de l'origine d'un produit carné comportant le prélèvement d'un échantillon de tissu animal avant que l'identité du vivant d'un animal soit perdue, le stockage de l'échantillon pour la durée de vie du produit carné, le génotypage du produit carné et d'échantillons stockés, et la comparaison du génotype du produit carné avec les génotypes d'échantillons stockés pour identifier l'origine du produit carné.

2. Un procédé tel que revendiqué dans la revendication 1 **caractérisé en ce que** l'animal est un animal de ferme domestiqué.

3. Un procédé tel que revendiqué dans la revendication 1 ou la revendication 2 **caractérisé en ce que** le prélèvement d'échantillon sur l'animal est fait à un seul point dans la chaîne de traitement.

4. Un procédé tel que revendiqué dans la revendication 3 **caractérisé en ce que** le prélèvement d'échantillon de tissu est effectué à un abattoir.

5. Un procédé tel que revendiqué dans la revendication 3 ou la revendication 4 **caractérisé en ce que** le prélèvement d'échantillon de tissu est effectué peu après le démembrement de la carcasse animale.

6. Un procédé tel que revendiqué dans n'importe lesquelles des revendications 3 à 5 **caractérisé en ce que** le prélèvement d'échantillon de tissu est effectué au point où les carcasses sont mises en lots pour un client de détail.

7. Un procédé tel que revendiqué dans la revendication 6 **caractérisé en ce qu'**un échantillon de viande est prélèvé sur chaque carcasse et marqué d'un numéro de tuerie interne propre à l'usine ou équivalent.

8. Un procédé tel que revendiqué dans les revendications 1 à 4 **caractérisé en ce que** le produit carné est un produit de boeuf.

9. Un procédé tel que revendiqué dans n'importe lesquelles des revendications 1 à 7 comportant le prélèvement d'un échantillon de tissu animal, l'extraction de matériau génétique du tissu animal et la réalisation d'une analyse génétique moléculaire sur le matériau génétique extrait.

10. Un procédé tel que revendiqué dans la revendication 9 **caractérisé en ce que** le matériau génétique comporte de l'ADN, l'ADN est amplifié à la suite de l'extraction et l'amplification comporte une réaction en chaîne de la polymérase.

11. Un procédé tel que revendiqué dans n'importe lesquelles des revendications 1 à 10 **caractérisé en ce que** l'échantillon est sélectionné dans le groupe comportant des racines de poil, de la peau, des frottis buccaux, du sang, du muscle, de l'os et n'importe quels organes internes et de préférence l'échantillon comporte un volume ou une taille unitaire et le prélèvement d'échantillon est réalisé à l'aide d'un dispositif de prélèvement d'échantillon dédié à la récupération de volume ou de taille unitaire.

12. Un procédé tel que revendiqué dans la revendication 10 ou la revendication 11 **caractérisé en ce que** l'ADN est extrait en utilisant l'extraction par alcali, l'alcali comporte NaOH ou KOH et l'ADN est extrait en ajoutant un volume unitaire d'alcali à l'échantillon, en chauffant l'échantillon pour extraire l'ADN, et en neutralisant l'alcali et l'alcali est neutralisé avec un acide.

13. Un procédé tel que revendiqué dans la revendication 12 **caractérisé en ce que** l'acide comporte HCl.

14. Un procédé tel que revendiqué dans n'importe lesquelles des revendications 9 à 13 **caractérisé en ce qu'**un colorant est ajouté à l'échantillon pour visualiser l'échantillon et le colorant comporte du rouge de crésol.

15. Un procédé tel que revendiqué dans n'importe lesquelles des revendications 12 à 14 **caractérisé en ce que** le pH de l'échantillon est au moins pH 8.

16. Un procédé tel que revendiqué dans n'importe lesquelles des revendications 9 à 15 **caractérisé en ce que** le tissu d'échantillon est placé dans une cellule d'identification et l'extraction est réalisée dans ladite cellule d'identification.

17. Un procédé tel que revendiqué dans la revendication 16 **caractérisé en ce que** la cellule d'identification est marquée de l'identification de l'animal ou de la viande à la suite du prélèvement d'échantillon et un numéro de référence de plaque de microtitrage de cellule d'identification est enregistré sur un ordinateur et le numéro de référence sur l'ordinateur peut être recherché.

18. Un procédé tel que revendiqué dans n'importe lesquelles des revendications 1 à 17 **caractérisé en ce que** le génotype de la viande génotypée est rentré sur un ordinateur et le génotype peut être recherché.

19. Un procédé tel que revendiqué dans la revendication 18 **caractérisé en ce que** le génotype est codé.
